(19) Europäisches Patentamt
European Patent Office
Office européen des brevets

(11) EP 1 595 524 B1

(12) # FASCICULE DE BREVET EUROPEEN

(45) Date de publication et mention de la délivrance du brevet:
**11.05.2011 Bulletin 2011/19**

(51) Int Cl.:
***A61K 8/81*** *(2006.01)* ***A61K 8/34*** *(2006.01)*
***A61K 8/42*** *(2006.01)* ***A61Q 3/02*** *(2006.01)*

(21) Numéro de dépôt: **05290547.8**

(22) Date de dépôt: **11.03.2005**

(54) **Composition de vernis à ongles comprenant un polymère séquencé et un agent plastifiant**

Nagellackzusammensetzung enthaltend einen Blockpolymer und einen Weichmacher

Nail composition comprising a sequenced polymer and a plastifying agent

(84) Etats contractants désignés:
**AT BE BG CH CY CZ DE DK EE ES FI FR GB GR HU IE IS IT LI LT LU MC NL PL PT RO SE SI SK TR**

(30) Priorité: **23.03.2004 FR 0450572**

(43) Date de publication de la demande:
**16.11.2005 Bulletin 2005/46**

(73) Titulaire: **L'Oréal**
**75008 Paris (FR)**

(72) Inventeurs:
- **Ilekti, Philippe**
  **94700 Maison-Alfort (FR)**
- **Leuridan, Frédéric**
  **75005 Paris (FR)**

(74) Mandataire: **Boulard, Denis**
**L'Oréal**
**D.I.P.I.**
**25-29 Quai Aulagnier**
**92600 Asnières (FR)**

(56) Documents cités:
**WO-A-02/45663**
**WO-A-99/39680**
**FR-A- 2 791 987**
**FR-A- 2 809 306**
**FR-A- 2 832 719**
**FR-A- 2 832 720**
**FR-A- 2 834 458**
**US-A- 5 681 877**
**US-A- 6 106 820**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 595 524 B1

## Description

La présente invention a pour objet un vernis à ongles comprenant un polymère séquencé et au moins un agent plastifiant particulier. L'invention a également pour objet un procédé de maquillage ou de soin des ongles.
La composition de vernis à ongles peut être employée comme base pour vernis ou "base-coat" en terminologie anglo-saxonne, comme produit de maquillage des ongles, comme composition de finition, encore appelée "top-coat", à appliquer sur le produit de maquillage des ongles ou bien encore comme produit de soin cosmétique des ongles. Ces compositions peuvent s'appliquer sur les ongles d'êtres humains ou bien encore sur des faux ongles.
On connaît des compositions à appliquer par exemple sur l'ongle, de type vernis à ongles ou base de soin pour ongles en milieu solvant, comprenant de manière usuelle, au moins un polymère filmogène, éventuellement un agent plastifiant, des pigments, des agents rhéologiques et des solvants.
Actuellement, la nitrocellulose reste encore le filmogène principal le plus utilisé dans les vernis à ongles à solvants dans des formulations à brillance et tenue optimisées.
Les formulations comprenant des nitrocelluloses permettent d'obtenir des films avec un niveau de dureté et de brillance corrects mais qui manquent d'adhérence sur l'ongle.
On peut remédier à cet inconvénient, en ajoutant des plastifiants mais, dans ce cas, il faut utiliser des quantités très importantes de plastifiants et de co-résines, de l'ordre de celles de la nitrocellulose.
Les recherches effectuées pour remplacer la nitrocellulose par d'autres agents filmogènes tels que des polyacryliques et des polyuréthanes dans les vernis à ongles, comme par exemple les dispersions aqueuses de polyuréthanes décrits dans le document EP0648485, n'ont pas donné de résultats satisfaisant notamment en termes de tenue et de résistance aux facteurs externes tels que l'eau ou les détergents.
En particulier, la sensibilité à l'eau du film de vernis accelère la dégradation du film de vernis sur les ongles, soit parce que le film peut se redissoudre dans l'eau, soit parce que le film se ternit en présence d'eau, soit parce que le film se décolle dans l'eau, soit encore parce que le film blanchit sous l'action de l'eau.
Le demandeur a découvert de façon surprenante que l'association d'un polymère séquencé et d'au moins un agent plastifiant particulier permet :

- une plastification des films sans recourir à l'adjonction de grandes quantités de plastifiants externes, tout en maintenant un bon niveau de dureté des films et
- une bonne résistance des vernis sur l'ongle aux chocs et/ou à l'écaillage ainsi qu'une bonne résistance à l'eau et donc une amélioration de la tenue dans le temps des vernis sur l'ongle. En particulier la sélection de plastifiants permet de diminuer la reprise en eau (sensibilité à l'eau) des films de vernis et donc d'augmenter leur résistance à l'eau.

L'invention a pour objet une composition de vernis à ongles comprenant, dans un milieu cosmétiquement acceptable comprenant un milieu solvant organique, au moins un polymère séquencé particulier et au moins un agent plastifiant choisi parmi les esters issus de la réaction d'un acide carboxylique avec un diol, les polyéthers, les diméthicone copolyol, l'éthyltosylamide et leurs mélanges.
Par "milieu cosmétiquement acceptable", on entend au sens de l'invention un milieu non toxique et susceptible d'être appliqué sur la peau, les phanères ou les lèvres du visage d'êtres humains.
L'invention a aussi pour objet un procédé cosmétique de maquillage ou de soin non thérapeutique des ongles comprenant l'application sur les ongles d'au moins une couche de la composition de vernis à ongles telle que définie ci-dessus.
L'invention a encore pour objet l'utilisation d'une composition de vernis à ongles comprenant au moins un polymère séquencé particulier et au moins un agent plastifiant choisi parmi les esters issus de la réaction d'un acide carboxylique avec un diol, les polyéthers, les diméthicone copolyol, l'éthyltosylamide et leurs mélanges, pour obtenir un film, déposé sur les ongles, brillant, de bonne tenue et résistant à l'eau.
Ce polymère séquencé peut être formulé comme seul polymère filmogène ou en complément d'un polymère filmogène classique comme la nitrocellulose ou un dérivé de cellulose, sans avoir l'inconvénient dans ce dernier cas, de l'adjonction de grandes quantités de plastifiants.

1) Polymère séquencé

Le polymère séquencé de la composition selon l'invention est avantageusement un polymère éthylénique séquencé linéaire filmogène
Par polymère "éthylénique" , on entend un polymère obtenu par polymérisation de monomères comprenant une insaturation éthylénique.
Par polymère "séquencé", on entend un polymère comprenant au moins 2 séquences distinctes, de préférence

au moins 3 séquences distinctes.

Le polymère est un polymère à structure linéaire. Par opposition, un polymère a structure non linéaire est, par exemple, un polymère à structure ramifiée, en étoile, greffée, ou autre.

Par polymère "filmogène", on entend un polymère apte à former à lui seul ou en présence d'un agent auxiliaire de filmification, un film continu et adhérent sur un support, notamment sur les matières kératiniques.

Le polymère séquencé de la composition selon la présente invention comprend au moins une première séquence et au moins une deuxième séquence de températures de transition vitreuses (Tg) différentes, lesdites première et deuxième séquences étant reliées entre elles par un segment intermédiaire comprenant au moins un monomère constitutif de la première séquence et au moins un monomère constitutif de la deuxième séquence.

Le segment intermédiaire est une séquence comprenant au moins un monomère constitutif de la première séquence et au moins un monomère constitutif de la deuxième séquence du polymère permet de "compatibiliser" ces séquences.

De façon préférentielle, le polymère séquencé ne comprend pas d'atomes de silicium dans son squelette. Par "squelette", on entend la chaîne principale du polymère, par opposition aux chaînes latérales pendantes.

De préférence, le polymère séquencé de la composition selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1 % en poids, à température ambiante (25°C).

De préférence, le polymère selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.
De manière plus spécifique, par "polymère non élastomère" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50.

Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.
On obtient alors un film d'environ 100 µm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.
Les éprouvettes sont étirées à une vitesse de 50 mm/min et la distance entre les mors est de 50 mm, ce qui correspond à la longueur initiale ($l_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($l_0$)
- on relâche la contrainte en imposant une vitesse de retour égale à la vitesse de traction, soit 50 mm/min et on mesure l'allongement résiduel de l'éprouvette en pourcentage, après retour à contrainte charge nulle ($\varepsilon_i$).

La recouvrance instantanée en % ($R_i$) est donnée par la formule ci-après:

$$R_i = (\varepsilon_{max} - \varepsilon_i) / \varepsilon_{max}) \times 100$$

Pour déterminer la recouvrance retardée, on mesure après 2 heures le taux d'allongement résiduel de l'éprouvette en pourcentage ($\varepsilon_{2h}$), 2 heures après retour à la contrainte charge nulle.

La recouvrance retardée après 2h en % ($R_{2h}$) est donnée par la formule ci-après:

$$R_{2h} = (\varepsilon_{max} - \varepsilon_{2h}) / \varepsilon_{max}) \times 100$$

A titre purement indicatif, le polymère selon un mode de réalisation de l'invention possède de préférence une recouvrance instantanée $R_i$ de 10% et une recouvrance retardée $R_{2h}$ de 30%.

Le polymère selon l'invention comprend au moins une première séquence (ou bloc) et au moins une deuxième séquence (ou bloc) ayant des températures de transition vitreuse (Tg) différentes, lesdites première et deuxième séquences étant reliées entre elles par un segment intermédiaire comprenant au moins un monomère constitutif de la première séquence et au moins un monomère constitutif de la deuxième séquence.

On précise que dans ce qui précède et ce qui suit les termes "première" et "deuxième" séquences ne conditionnent nullement l'ordre desdites séquences (ou blocs) dans la structure du polymère.

Avantageusement, le polymère séquencé de la composition selon l'invention a un indice de polydispersité I supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère selon l'invention est de préférence inférieure ou égale à 300 000, elle va par exemple de 35 000 à 200 000, et mieux de 45 000 à 150 000.

La masse moyenne en nombre (Mn) du polymère selon l'invention est de préférence inférieure ou égale à 70 000, elle va par exemple de 10 000 à 60 000, et mieux de 12 000 à 50 000.

Chaque séquence ou bloc du polymère séquencé de la composition selon l'invention est issue d'un type de monomère ou de plusieurs types de monomères différents.

Cela signifie que chaque séquence peut être constituée d'un homopolymère ou d'un copolymère ; ce copolymère constituant la séquence pouvant être à son tour statistique ou alterné.

Avantageusement, le segment intermédiaire comprenant au moins un monomère constitutif de la première séquence et au moins un monomère constitutif de la deuxième séquence du polymère est un polymère statistique.

De préférence, la séquence intermédiaire est issue essentiellement de monomères constitutifs de la première séquence et de la deuxième séquence.

Par "essentiellement", on entend au moins à 85%, de préférence au moins à 90%, mieux à 95% et encore mieux à 100%.

Avantageusement, la séquence intermédiaire du polymère séquencé a une température de transition vitreuse Tg comprise entre les températures de transition vitreuse des première et deuxième séquences.

Les première et deuxième séquences du polymère séquencé de la composition ont des températures de transition vitreuse différentes.

Les températures de transition vitreuse indiquées des première et deuxième séquences du polymère séquencé peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_{i} (\varpi_i / Tg_i) ,$$

$\varpi_i$ étant la fraction massique du monomère i dans la séquence considerée et $Tg_i$ étant la température de transition vitreuse de l'homopolymère du monomère i.

Sauf indication contraire, les Tg indiquées pour les première et deuxième séquences du polymère séquencé dans la présente demande sont des Tg théoriques.

La première séquence du polymère séquencé est une séquence ayant une Tg supérieure ou égale à 40°C et la deuxième séquence est une séquence ayant une Tg inférieure ou égale à 26°C.

a) Séquence ayant une Tg supérieure ou égale à 40°C

La séquence ayant une Tg supérieure ou égale à 40°C du polymère séquencé a par exemple une Tg allant de 40 à 150°C, de préférence supérieure ou égale à 50°C, allant par exemple de 50°C à 120°C, et mieux supérieure ou égale à 60°C, allant par exemple de 60°C à 120°C .

La séquence ayant une Tg supérieure ou égale à 40°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de

l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20 à 40°C et/ou les monomères ayant une Tg inférieure ou égale à 20°C, par exemple une Tg allant de -100 à 20°C, de préférence inférieure à 15°C, notamment allant de - 80°C à 15°C et mieux inférieur à 10°C, par exemple allant de -50°C à 0°C à, tels que décrits plus loin, .

Les monomères dont les homopolymères ont une température de transition vitreuse supérieure ou égale à 40°C sont choisis parmi les monomères suivants, appelés aussi monomères principaux :

- les méthacrylates de formule $CH_2 = C(CH_3)-COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH-COOR_2$
  dans laquelle $R_2$ repré sente un groupe cycloalkyle en $C_4$ à $C_{12}$ tel que l'acrylate d'isobornyle ou un groupe tertio butyle,
- et leurs mélanges.

Des monomères principaux particulièrement préférés sont le méthacrylate de méthyle, le (méth)acrylate d'isobutyle, le (méth)acrylate d'isobornyle et leurs mélanges.

<u>b) Séquence avant une Tg inférieure ou égale à 20°C</u>

La séquence ayant une Tg inférieure ou égale à 20°C du polymère séquencé a par exemple une Tg allant de -100 à 20°C, de préférence inférieure ou égale à 15°C, notamment allant de -80°C à 15°C et mieux inférieure ou égale à 10°C, par exemple allant de -50°C à 0°C.

La séquence ayant une Tg inférieure ou égale à 20°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).

Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.
Elle peut par exemple comprendre

- un ou plusieurs monomères dont l'homopolymère correspondant a une Tg inférieure ou égale à 20°C, par exemple une Tg allant de -100°C à 20 °C, de préférence inférieure à 15°C, notamment allant de - 80°C à 15°C et mieux inférieur à 10°C, par exemple allant de -50°C à 0°C et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.

Les monomères dont l'homopolymère a une Tg inférieure ou égale à 20°C sont choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2 = C(CH_3)$-$COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.
Chacune des séquences du polymère séquence peut néanmoins contenir en proportion minoritaire au moins un monomère constitutif de l'autre séquence.
Ainsi la première séquence du polymère séquencé peut contenir au moins un monomère constitutif de la deuxième séquence et inversement.
Chacune des première et/ou deuxième séquence du polymère séquencé, peu(ven)t comprendre, outre les monomères indiqués ci-dessus, un ou plusieurs autres monomères appelés monomères additionnels, différents des monomères principaux cités précédemment.
La nature et la quantité de ce ou ces monomères additionnels sont choisies de manière à ce que la séquence dans laquelle ils se trouvent ait la température de transition vitreuse désirée.
Ce monomère additionnel est par exemple choisi parmi :

a) les monomères hydrophiles tels que :

- les monomères à insaturation(s) éthylénique(s) comprenant au moins une fonction acide carboxylique ou sulfonique comme par exemple :

  l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)$-$COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)$-$COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthylèné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris ( triméthylsiloxy ) silane,

- et leurs mélanges.

Des monomères additionnels particulièrement préférés sont l'acide acrylique, l'acide méthacrylique, le méthacrylate de trifluoroéthyle et leurs mélanges.
Selon un mode préféré de réalisation, le polymère séquencé de la composition selon l'invention est un polymère non siliconé, c'est à dire un polymère exempt d'atome de silicium.
Ce ou ces monomères additionnels représente(nt) généralement une quantité inférieure ou égale à 30% en

poids, par exemple de 1 à 30% en poids, de préférence de 5 à 20% en poids et, de préférence encore, de 7 à 15% en poids du poids total des première et/ou deuxième séquences.

De préférence, chacune des première et deuxième séquences comprend au moins un monomère choisi parmi les esters d'acide (méth)acrylique, et éventuellement au moins un monomère choisi parmi l'acide (méth)acrylique, et leurs mélanges.

Avantageusement, chacune des première et deuxième séquences est issue en totalité d'au moins un monomère choisi parmi l'acide acrylique, les esters d'acide (méth)acrylique, et éventuellement d'au moins un monomère choisi parmi l'acide (méth)acrylique, et leurs mélanges

De préférence, le polymère séquencé de la composition selon l'invention est exempt de styrène. Par "polymère exempt de styrène", on entend un polymère contenant moins de 10 % en poids, par rapport au poids total du polymère, de préférence moins de 5 % en poids, mieux moins de 2 % en poids, mieux moins de 1 % en poids, voire ne contenant pas, de monomère styrénique comme le styrène, les dérivés de styrène tels que le méthylstyrène, le chlorostyrène ou le chlorométhylstyrène. de styrène ou de dérivés du styrène comme par exemple le méthylstyrène, le chlorostyrène ou le chlorométhylstyrène.

Le polymère séquencé de la composition selon l'invention peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120°C),
- une fois cette température atteinte, les monomères constitutifs de la première séquence sont introduits en présence d'une partie de l'initiateur de polymérisation,
- au bout d'un temps T correspondant à un taux de conversion maximum de 90%, les monomères constitutifs de la deuxième séquence et l'autre partie de l'initiateur sont introduits,
- on laisse réagir le mélange pendant un temps T' ( allant de 3 à 6 h) au bout duquel le mélange est ramené à température ambiante,
- on obtient le polymère en solution dans le solvant de polymérisation.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.

Le polymère séquencé de la composition selon l'invention comprend au moins une (notamment une) première séquence ayant une Tg supérieure ou égale à 40°C, telle que décrite plus haut au a) et au moins une (notamment une) deuxième séquence ayant une Tg inférieure ou égale à 20°C, telle que décrite plus haut au b).

De préférence, la première séquence ayant une Tg supérieure ou égale à 40°C du polymère séquencé est un copolymère issu de monomères qui sont tels que l'homopolymère préparé à partir de ces monomères a une température de transition vitreuse supérieure ou égale à 40°C, tels que les monomère décrits plus haut.

Avantageusement, la deuxième séquence ayant une Tg inférieure ou égale à 20°C est un homopolymère issu de monomères qui sont tel(s) que l'homopolymère préparé à partir de ces monomères a une température de transition vitreuse inférieure ou égale à 20°C, tels que les monomères décrits plus haut.

De préférence, la proportion de la séquence ayant une Tg supérieure ou égale à 40°C du polymère séquencé va de 20 à 90% en poids du polymère, mieux de 30 à 80% et encore mieux de 50 à 70%.

De préférence, la proportion de la séquence ayant une Tg inférieure ou égale à 20°C va de 5 à 75% en poids du polymère, de préférence de 15 à 50% et mieux de 25 à 45%.

Ainsi, selon une première variante, le polymère séquencé de la composition selon l'invention peut comprendre :

- une première séquence de Tg supérieure ou égale à 40°C, par exemple ayant une Tg allant de 70 à 110°C, qui est un copolymère méthacrylate de méthyle / acide acrylique,
- une deuxième séquence de Tg inférieure ou égale à 20°C, par exemple allant de 0 à 20°C, qui est un homopolymère d'acrylate de méthyle et
- une séquence intermédiaire qui est un copolymère méthacrylate de méthyle/acide acrylique/acrylate de méthyle.

Selon une seconde variante, le polymère séquencé de la composition selon l'invention peut comprendre :

- une première séquence de Tg supérieure ou égale à 40°C, par exemple allant de 70 à 100°C, qui est un copolymère méthacrylate de méthyle/acide acrylique/méthacrylate de trifluoroéthyle,
- une deuxième séquence de Tg inférieure ou égale à 20°C, par exemple allant de 0 à 20°C, qui est un homopolymère d'acrylate de méthyle et

- une séquence intermédiaire qui est un copolymère statistique méthacrylate de méthyl/acide acrylique/acrylate de méthyle/méthacrylate de trifluoroéthyle.

Milieu solvant organique

Le milieu cosmétiquement acceptable de la composition cosmétique selon l'invention comprend un milieu solvant organique comprenant un solvant organique ou un mélange de solvants organiques.
Le solvant organique peut être choisi parmi :

- les cétones liquides à température ambiante tels que méthyléthylcétone, méthylisobutylcétone, düsobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle ,
- les acétals tels que méthylal ;
- et leurs mélanges.

De préférence, le solvant est choisi parmi les esters à chaîne courte ayant de 3 à 8 atomes de carbone au total, tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle et leurs mélanges.
Le milieu solvant organique peut représenter de 10 à 95% en poids, par rapport au poids total de la composition, de préférence de 15% à 80% en poids, et mieux de 20 à 60% en poids.
Le milieu cosmétiquement acceptable de la composition selon l'invention peut éventuellement comprendre un milieu aqueux.

Agent plastifiant

L'agent plastifiant est choisi parmi les esters issus de la réaction d'un acide carboxylique avec un diol, les polyéthers, les dimethicone copolyols, l'éthyltosylamide et leurs mélanges.
Les esters issus de la réaction d'un acide carboxylique avec un diol sont de préférence des monoesters issus de la réaction d'un acide monocarboxylique de formule $R_{11}COOH$ avec un diol de formule $HOR_{12}0H$ avec $R_{11}$ et $R_{12}$, identiques ou différents, représentent une chaîne hydrocarbonée, comprenant de préférence de 3 à 15 atomes de carbone, linéaire, ramifiée ou cyclique, saturée ou insaturée comprenant éventuellement un ou plusieurs hétéroatomes tels que N, O , S.
De préférence, $R_{11}$ est un radical alkyle en $C_3$-$C_5$ tel que le propyle, le butyle, l'isobutyle et $R_{12}$ est une chaîne hydrocarbonée linéaire saturée comprenant de 5 à 10 atomes de carbone.
Avantageusement, l'agent plastifiant est un monoester résultant de la réaction de l'acide isobutyrique et d'octanediol tel que le triméthyl-2,2,4 pentane diol 1,3, comme le TEXANOL Ester Alcohol commercialisé par la société Eastman Chemical.
Les polyéthers sont de préférence choisi parmi ceux qui présentent la formule générale suivante $H\text{-}(O\text{-}C_xH2_x)m\text{-}(O\text{-}C_yH2_y)n\text{-}OH$ (I) dans laquelle x et y, identiques ou différents, sont des entiers allant de 2 à 10, de préférence de 3 à 5 et
m et n sont indépendamment des entiers allant de 0 à 1000, de préférence de 0 à 100.
De préférence, x est égale à 3 et y=0.
A titre de polyéthers répondant à cette formule, on peut citer les polyéthylène glycols, les polypropylène glycols, les copolymères polyéthylène glycols-polypropylène glycols et leurs mélanges. On utilise avantageusement des polypropylène glycols de haut poids moléculaire, ayant par exemple une masse moléculaire allant de 500 à 15000, de préférence

de 600 à 10 000, et en particulier les polypropylène glycols présentant une masse moléculaire égale à 2000 ou 4000.

L'agent plastifiant peut également être choisi parmi les dimethicone copolyols.

Par dimethicone copolyol, on entend un polymère de polydiméthylsiloxane comprenant des groupes polyéthers, tel que polyoxyethylène ou polyoxypropylène, de préférence polyoxypropylène, pendants ou en bouts de chaine.

De préférence, le dimethicone copolyol selon l'invention ne comprend pas de groupement alkyle à longue chaîne de plus de 8 atomes de carbone, notamment en C8-C22.

On peut notamment utiliser comme diméthicone copolyols ceux répondant à la formule (II) suivante :

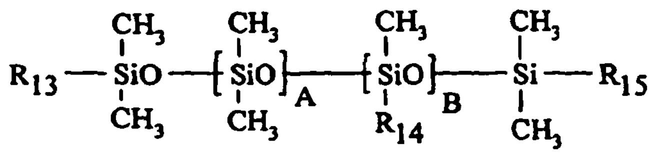

(II)

dans laquelle :

- $R_{13}$, $R_{14}$ et $R_{15}$, représentent indépendamment :

- un radical alkyle en C1-C6, tel que méthyle, éthyle, propyle, butyle, pentyle ou hexyle ou
- un groupement $-(CH2)a - (O-C_xH2_x)m-(O-C_yH2_y)n- O-R_{16}$ dans lequel

    - a est un nombre entier allant de 0 à 8,
    - $R_{16}$ représente un atome d'hydrogène ou un radical alkyle en C1-C6, tel que méthyle, éthyle, propyle, butyle, pentyle ou hexyle, avec la condition que l'un au moins des radicaux $R_{13}$, $R_{14}$ et $R_{15}$, est un groupement $-(CH2) a - (O-C_xH2_x)m-(O-C_yH2_y)n -O-R_{16}$ tel que défini ci-dessus,
    - x et y, identiques ou différents, sont des entiers allant de 2 à 10, de préférence de 3 à 5, et mieux, x=3 et y=0,
    - m et n sont indépendamment des entiers allant de 0 à 1000, de préférence de 0 à 100,

- A est un nombre entier allant de 0 à 200 ;
- B est un nombre entier allant de 0 à 100 ; à la condition que A et B ne soient pas simultanément égaux à zéro.

Selon un mode de réalisation particulier de l'invention, le dimethicone copolyol est choisi parmi les composés de formule (II) pour lesquels

- B=0,
- A est un entier allant de 0 à 200,
- $R_{13}$ et $R_{15}$ sont identiques et représentent un radical $-(CH2)a - (O-C_xH2_x)m-(O-C_yH2_y)n-O-R_{16}$ dans lequel a est un nombre entier allant de 0 à 8, $R_{16}$ représente un atome d'hydrogène ou un radical alkyle en C1-C6, x=3 et y=0 et m et n sont indépendamment des entiers allant de 0 à 1000, de préférence de 0 à 100.

On peut citer en particulier les dimethicone copolyols à groupements $\alpha$-$\omega$ propyl polyoxypropylène tel que le dimethicone copolyol commercialisé sous la référence SILWET L-8500 par la société OSI et MAZIL.

On peut également utiliser comme agent plastifiant l'éthyltosylamide tel que commercialisé sous la référence RESIMPOL 8 par la société Pan-Americana.

Le ou les agents plastifiants peu(ven)t être présent(s) en une quantité allant de 1 à 15% en poids par rapport au poids total de la composition, de préférence de 2 à 10%, et mieux de 3 à 8% en poids.

<u>Polymère filmogène additionnel</u>

La composition peut comprendre, outre le polymère séquencé de la composition selon l'invention, un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose,

l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Le polymère filmogène additionnel peut être présent dans la composition selon l'invention en une teneur allant de 0,1 % à 60 % en poids, par rapport au poids total de la composition, de préférence allant de 2 % à 40 % en poids, et mieux de 5 % à 25 % en poids.

Matière colorante

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Autres Additifs

La composition peut comprendre, en outre, d'autres ingrédients utilisés couramment dans les compositions cosmétiques. De tels ingrédients peuvent être choisis parmi les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant : i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

Les exemples qui suivent illustrent de manière non limitative l'invention.

Dans les exemples qui suivent, les Tg indiquées pour les première et deuxième séquences sont des Tg théoriques calculées de la manière définie précédemment.

**Exemple 1 : Préparation d'un polymère de poly(méthacrylate de méthyle/acide acrylique/acrylate de méthyle)**

100 g d'acétate de butyle sont introduits dans un réacteur de 1 litre, puis on augmente la température de façon à passer de la température ambiante (25°C) à 90°C en 1 heure.

On ajoute ensuite, à 90°C et en 1 heure, 180 g de méthacrylate de méthyle, 30 g d'acide acrylique, 40 g d'acétate de butyle, 70 g d'isopropanol et 1,8 g de 2.5- Bis(2-éthylhexanoylperoxy)-2.5-diméthylhexane (Trigonox® 141 d'Akzo Nobel).

Le mélange est maintenu 1 heure à 90°C.

On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.

Le mélange est maintenu 3 heures à 90°C, puis dilué par 105 g d'acétate de butyle et 45 g d'isopropanol, puis l'ensemble est refroidi.

On obtient une solution à 40% de matière active en polymère dans le mélange acétate de butyle / isopropanol.

On obtient un polymère comprenant une première séquence ou bloc poly (méthacrylate de méthyle/acide acrylique) ayant une Tg de 100°C une deuxième séquence ou bloc polyacrylate de méthyle ayant une Tg de 10°C et une séquence intermédiaire qui est un polymère statistique méthacrylate de méthyle/acide acrylique/polyacrylate de méthyle.

Ce polymère présente une masse moyenne en poids de 52000 et une masse moyenne en nombre de 18000, soit un indice de polydispersité I de 2,89

**Exemple 2 : Préparation d'un polymère de poly (méthacrylate de méthyle /acide acryliquelacrylate de méthyle)**

100 g d'acétate de butyle sont introduits dans un réacteur de 1 litre, puis on augmente la température de façon à passer de la température ambiante (25°C) à 90°C en 1 heure.

On ajoute ensuite, à 90°C et en 1 heure, 150g de méthacrylate de méthyle, 30 g d'acide acrylique, 30 g d'acrylate de méthyle, 40 g d'acétate de butyle, 70 g d'isopropanol et 1,8 g de 2.5- Bis(2-éthylhexanoylperoxy)-2.5-diméthylhexane (Trigonox® 141 d'Akzo Nobel).

Le mélange est maintenu 1 heure à 90°C.

On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.

Le mélange est maintenu 3 heures à 90°C, puis dilué par 105 g d'acétate de butyle et 45 g d'isopropanol, puis l'ensemble est refroidi.

On obtient une solution à 40% de matière active en polymère dans le mélange acétate de butyle / isopropanol.

On obtient un polymère comprenant une première séquence ou bloc poly (acide acrylique/acrylate de méthyle) ayant une Tg de 80°C, une deuxième séquence polyacrylate de méthyle ayant une Tg de 10°C et une séquence intermédiaire qui est un polymère statistique acide acryliquelacrylate de méthyle/polyacrylate de méthyle.

Ce polymère présente une masse moyenne en poids de 50 000 et une masse moyenne en nombre de 17 000, soit un indice de polydispersité I de 2,95.

**Exemple 3 : Préparation d'un polymère de polylacide acrylique/acrylate de méthyle/acrylate de méthyle/méthacrylate de trifluoroéthyle)**

100 g d'acétate de butyle sont introduits dans un réacteur de 1 litre, puis on augmente la température de façon à passer de la température ambiante (25°C) à 90°C en 1 heure.
On ajoute ensuite, à 90°C et en 1 heure, 120 g de méthacrylate de méthyle, 30 g d'acide acrylique, 60 g de méthacrylate de trifluoroéthyle, 40 g d'acétate de butyle, 70 g d'isopropanol et 1,8 g de 2.5- Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
On obtient un polymère comprenant une première séquence ou bloc poly (acide acrylique/méthacrylate de méthyle/méthacrylate de trifluoroéthyle) ayant une Tg de 85°C, une deuxième séquence polyacrylate de méthyle ayant une Tg de 10°C et une séquence intermédiaire qui est un polymère statistique acide acrylique/acrylate de méthyle/polyacrylate de méthyle/méthacrylate de trifluoroéthyle.
Ce polymère présente une masse moyenne en poids de 53 000 et une masse moyenne en nombre de 17 500, soit un indice de polydispersité I de 3,03.

**Exemple 4 : Préparation d'un polymère de poly(méthacrylate de méthyle/acrylate de méthyle/acide acrylique)**

210 g d'acétate d'éthyle sont introduits dans un réacteur de 1 litre, puis on augmente la température de façon à passer de la température ambiante (25°C) à 78°C en 1 heure.
On ajoute ensuite, à 78°C et en 1 heure, 54 g de méthacrylate de méthyle, 21 g d'acide acrylique, 135 g d'acrylate de méthyle et 1,8 g de 2.5-Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 78°C et en 1 heure, 90 g de méthacrylate de méthyle, 90 g d'acétate d'éthyle et 1,2 g de 2.5-Bis(2-éthylhexanoylperoxy)-2.5-diméthylhexane.
Le mélange est maintenu 3 heures à 78°C, puis dilué par 150 g d'acétate d'éthyle puis l'ensemble est refroidi.
On obtient une solution à 40% de matière active en polymère dans l'acétate d'éthyle.
Le polymère obtenu comprend une première séquence ou bloc poly(acrylate de méthyle/méthacrylate de méthyle/acide acrylique) ayant une Tg de 35°C une deuxième séquence ou bloc poly (méthacrylate de méthyle ) ayant une Tg de 100°C et une séquence intermédiaire qui est un polymère statistique méthacrylate de méthyle/acide acrylique/polyacrylate de méthyle.
Ce polymère présente une masse moyenne en poids de 141 000 et une masse moyenne en nombre de 50 000, soit un indice de polydispersité I de 2,82

**Exemple 5 : Préparation d'un polymère de poly(méthacrylate de méthyle/acrylate de méthyle/acide acrylique)**

100 g d'acétate de butyle sont introduits dans un réacteur de 1 litre, puis on augmente la température de façon à passer de la température ambiante (25°C) à 90°C en 1 heure. On ajoute ensuite, à 90°C et en 1 heure, 50,4 g de méthacrylate de méthyle, 21 g d'acide acrylique, 138,6 g d'acrylate de méthyle, 40 g d'acétate de butyle, 70 g d'isopropanol et 1,8 g de 2.5-Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g de méthacrylate de méthyle, 70 g d'acétate de butyle, 20 g d'isopropanol et 1,2 g de 2.5-Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
Le polymère obtenu comprend une première séquence ou bloc poly(acrylate de méthyle/méthacrylate de méthyle/acide acrylique) ayant une Tg de 35°C une deuxième séquence ou bloc poly (méthacrylate de méthyle ) ayant une Tg de 100°C et une séquence intermédiaire qui est un polymère statistique méthacrylate de méthyle/acide acrylique/polyacrylate de méthyle.

**Exemple 6 : Vernis à ongles**

On a préparé des vernis à ongles ayant les compositions suivantes : les exemples 1 à 4 comprennent un agent plastifiant selon l'invention, les exemples 5 et 6 comprennent un plastifiant selon l'art antérieur.

| | **Exemple 1** | **Exemple 2** | **Exemple 3** | **Exemple 4** | **Exemple 5** | **Exemple 6** |
|---|---|---|---|---|---|---|
| Polymère de l'exemple 3 (*MA) | 25 | 25 | 25 | 25 | 25 | 25 |
| Ester de l'acide isobutyrique et du triméthyl-2,2,4 pentane diol 1,3 (Texanol de Eastman Chemical) | 3 | | | | | |
| Polypropylène glycol (Mw = 2000) | | 3 | | | | |
| Dimethicone copolyol (SILWET L-8500 de OSI et MAZIL) | | | 3 | | | |
| Ethyl tosylamide | | | | 3 | | |
| Hexylène glycol | | | | | 3 | |
| Polyoxyethylènelpolyoxypropylène glycol | | | | | | 3 |
| Acétate d'éthyle | Qsp 100 | Qsp 100 | Qsp 100 | Qsp 100 | Qsp 100 | Qsp 100 |
| *MA= Matière Active | | | | | | |

Pour chacun des exemples, on a mesuré la reprise à l'eau du film de composition comme indiqué précédemment.

Les résultats sont présentés dans le tableau suivant :

| | **Reprise en eau** |
|---|---|
| Exemple 1 | 1,5 |
| Exemple 2 | 2,9 |
| Exemple 3 | 2 |
| Exemple 4 | 2,5 |
| Exemple 5 | 23,3 |
| Exemple 6 | 12 |

Les compositions 1 à 4 comprenant un agent plastifiant selon l'invention forment des films de vernis qui présentent une reprise en eau inférieure aux compositions de l'art antérieur, et présentent de ce fait une meilleure résistance à l'eau.

Les compositions 1 à 4 ont également été jugées comme étant brillantes et présentant une bonne tenue dans le temps.

**Revendications**

1. Composition de vernis à ongles comprenant, dans un milieu cosmétiquement acceptable comprenant un milieu solvant organique, au moins un polymère séquencé et au moins un agent plastifiant choisi parmi les esters issus de la réaction d'un acide carboxylique avec un diol, les polyéthers, les dimethicone copolyol, l'éthyltosylamide et leurs mélanges,
ledit polymère séquence comprenant au moins une première séquence et au moins une deuxième séquence, lesdits

première et deuxième séquences étant reliées entre elles par un segment intermédiaire comprenant au moins un monomère constitutif de la première séquence et au moins un monomère constitutif de la deuxième séquence,
la première séquence ayant une température de transition vitreuse (Tg) supérieure ou égale à 40°C, ladite première séquence étant issue en totalité ou en partie de un ou plusieurs monomères, qui sont tels que l'homopolymère préparé à partir de ces monomères a une température de transition vitreuse supérieure ou égale à 40°C, ou ladite première séquence étant un copolymère issu de monomères qui sont tels que l'homopolymère préparé à partir de ces monomères a une température de transition vitreuse supérieure ou égale à 40C,
les monomères dont l'homopolymère correspondant a une température de transition vitreuse supérieure ou égale à 40°C étant choisis parmi les monomères suivants :

- les méthacrylates de formule $CH_2 = C(CH_3)\text{-}COOR_1$
dans laquelle $R_1$ représente un groupe alkyle non substitué, alinéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2$= CH-$COOR_2$
dans laquelle $R_2$ représente un groupe cycloalkyle en $C_4$ à $C_{12}$ tel que l'acrylate d'isobornyle ou un groupe tertio butyle,
- et leurs mélanges,

et la deuxième séquence ayant une température de transition vitreuse inférieure ou égale à 20°C, ladite deuxième séquence étant un homopolymère issu de monomères qui sont tel(s) que l'homopolymère préparé à partir de ces monomères a une température de transition vitreuse inférieure ou égale à 20°C,
les monomères dont l'homopolymère correspondant a une température de transition vitreuse inférieure ou égale à 20°C étant choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O. N, S ;
- les méthacrylates de formule $CH_2 = C(CH_3\text{-}COOR_4$,
$R_4$ représentant un groupé alkyle non substitué en $C_6$ à $C_{12}$ linéaire ou ramifié, dans lequel se trouve(nt) éventuellement intercalé(s) un ou plusieurs hétéroatomes choisis parmi 0, N et S;
et leurs mélanges.

2. Composition selon la revendication précédente **caractérisée en ce que** le polymère séquence est un polymère éthylénique séquence linéaire filmogène.

3. Composition selon l'une des revendications 1 ou 2 **caractérisée en ce que** les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

4. Composition selon l'une des revendications1à 3 **caractérisée en ce que** la proportion de la première séquence du polymère séquencé va de 20 à 90% en poids du polymère, mieux de 30 à 80% et encore mieux de 50 à 70%.

5. Composition selon l'une des revendications 1 à 4 **caractérisée en ce que** les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

6. Composition selon l'une des revendications 1 à 5 **caractérisée en ce que** la proportion de la deuxième séquence ayant une Tg inférieure ou égale à 20°C du polymère séquence va de 5 à 75% en poids du polymère, mieux de 15 à 50% et encore mieux de 25 à 45%.

7. Composition selon l'une des revendications précédentes, **caractérisée en ce que** la première séquence et/ou la deuxième séquence du polymère séquencé comprend au moins un monomère additionnel.

8. Composition selon la revendication précédente, **caractérisée en ce que** le monomère additionnel est choisi parmi les monomères hydrophiles, les monomères à insaturation éthylénique comprenant un ou plusieurs atomes de silicium et leurs mélanges.

9. Composition selon la revendication 7 ou 8, **caractérisée en ce que** le monomère additionnel est choisi parmi :

- les monomères à insaturation(s) éthylénique(s) comprenant au moins une fonction acide carboxylique ou sulfonique
- les méthacrylates de formule $CH_2 = C(CH_3)\text{-}COOR_8$ dans laquelle $R_8$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle et les atomes d'halogènes,
- les méthacrylates de formule $CH_2 = C(CH_3)\text{-}COOR_9$, $R_9$ représentant un groupé alkyle en $C_8$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$, $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthylèné comprenant de 5 à 30 motifs d'oxyde d'éthylène
- les monomères à insaturation(s) éthylénique(s) comprenant au moins une fonction amine tertiaire, et leurs mélanges.

10. Composition selon l'une des revendications 7 à 9, **caractérisée en ce que** le ou les monomères additionnels sont choisis parmi l'acide acrylique, l'acide méthacrylique, le méthacrylate de trifluoroéthyle et leurs mélanges.

11. Composition selon l'une des revendications 7 à 10, **caractérisée en ce que** le ou les monomères additionnel(s) représente(nt) de 1 à 30% en poids du poids total des première et/ou deuxième séquences du polymère séquencé.

12. Composition selon l'une des revendications précédentes, **caractérisé en ce que** chacune des première et deuxième séquence du polymère séquencé comprend au moins un monomère choisi parmi les esters d'acide (meth)acrylique, et éventuellement au moins un monomère choisi parmi l'acide (meth)acrylique et leurs mélanges.

13. Composition selon l'une des revendications précédentes, **caractérisé en ce que** chacune des première et deuxième séquence du polymère séquence est issue en totalité d'au moins un monomère choisi parmi l'acide acrylique, les esters d'acide (méth)acylique, et éventuellement d'au moins un monomère choisi parmi l'acide (méth)acrylique, et leurs mélanges.

14. Composition selon rune des revendications précédente, **caractérisée en ce que** écart entre les températures de transition vitreuse (Tg) des première et deuxième séquences du polymère séquencé est supérieur à 30°C et mieux supérieure à 40°C.

15. Composition selon l'une des revendications précédentes, **caractérisée en ce que** le segment intermédiaire du polymère séquencé a une température de transition vitreuse comprise entre les températures de transition vitreuse des première et deuxième séquences.

16. Composition selon l'une des revendications précédente, **caractérisée en ce que** le polymère séquencé a un indice de polydispersité 1 supérieur à 2, de préférence supérieur ou égal à 2,5, de préférence supérieure ou égal à 2,8.

17. Composition selon l'une des revendications précédentes, **caractérisée en ce que** le polymère séquencé a un indice de polydispersité compris entre 2,8 et 6.

18. Composition selon l'une des revendications précédentes, **caractérisée en ce que** le polymère séquencé n'est pas soluble à une teneur en matière active d'au moins 1% en poids dans l'eau ou dans un mélange d'eau et de monoalcools inférieurs linéaires ou ramifiés ayant de 2 à 5 atomes de carbone, sans modification de pH, à température ambiante (25°C).

19. Composition selon l'une des revendications précédentes, **caractérisée en ce que** le polymère séquencé n'est pas un élastomère.

**20.** Composition selon l'une des revendications précédentes, **caractérisée en ce que** le polymère séquencé représente de 0,1 à 60% en poids en matière sèche par rapport au poids total de la composition, de préférence de 0,5 à 50% et mieux de 1 à 40% en poids.

**21.** Composition selon l'une des revendications précédentes, **caractérisée en ce que** le milieu solvant organique comprend au moins un solvant organique choisi parmi :

- les cétones liquides à température ambiante tels que les méthyléthylcétone, méthylisobutylcétone, diisobutylcétone, l'isophorone, la cyclohexanone l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle,
- les acétales tels que le méthylal ;
- leurs mélanges.

**22.** Composition selon l'une des revendications précédentes, **caractérisée en ce que** le milieu solvant organique représente de 10 à 95% en poids, par rapport au poids total de la composition, de préférence de 15% à 80% en poids, et mieux de 20 à 60% en poids.

**23.** Composition selon l'une des revendications 1 à 22, **caractérisée en ce que** l'ester d'acide carboxylique et de diol est un monoester.

**24.** Composition selon l'une des revendications 1 à 23, **caractérisée en ce que** l'ester d'acide carboxylique et de diol est issu de la réaction d'un acide monocarboxylique de formule $R_{11}COOH$ avec un diol de formule $HOR_{12}OH$ avec $R_{11}$ et $R_{12}$ identiques ou différents, représentent une chaîne hydrocarbonée comprenant de préférence de 3 à 15 atomes de carbone, linéaire, ramifiée ou cyclique, saturée ou insaturée comprenant éventuellement un ou plusieurs hétéroatomes tels que N, O, S.

**25.** Composition selon la revendication précédente, **caractérisée en ce que** $R_{11}$ est un radical alkyle en $C_3$-$C_5$ et $R_{12}$ est une chaîne hydrocarbonée linéaire saturée comprenant de 5 à 10 atomes de carbone.

**26.** Composition selon l'une des revendications précédentes, **caractérisée en ce que** l'agent plastifiant est un monoester résultant de la réaction de l'acide isobutyrique et du triméthyl-2,2,4 pentane diol 1,3.

**27.** Composition selon l'une des revendications précédentes, **caractérisée en ce que** les polyéthers présentent la formule générale suivante :

$$H\text{-}(O\text{-}C_xH2_x)m\text{-}(O\text{-}C_yH2_y)n\text{-}OH \qquad (I)$$

dans laquelle x et y, identiques ou différents, sont des entiers allant de 2 à 10, de préférence de 3 à 5 et m et n sont indépendamment des entiers allant de 0 à 1000, de préférence de 0 à 100.

**28.** Composition selon l'une des revendications précédentes, **caractérisée en ce que** les polyéthers sont choisis parmi les polyéthylène glycols, les polypropylène glycols, les copolymères polyéthylène glycols- polypropylène glycols et leurs mélanges.

**29.** Composition selon l'une des revendications précédentes, **caractérisée en ce que** les polyéthers sont choisis parmi

les polyéthylène glycols et les polypropylène glycols présentant une masse moléculaire allant de 500 à 15000, de préférence de 600 à 10000 et leurs mélanges.

**30.** Composition selon l'une des revendications précédentes, **caractérisée en ce que** les dimethicone copolyols répondent à la formule (II) suivante :

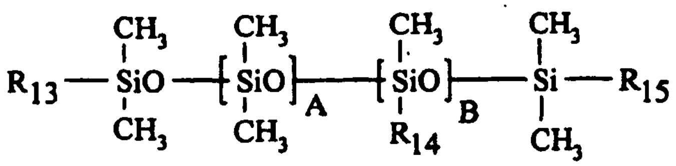

**(II)**

dans laquelle :

- $R_{13}$, $R_{14}$ et $R_{15}$, représentent indépendamment :
- un radical alkyle en C1-C6, tel que méthyle, éthyle, propyle, butyle, pentyle ou hexyle ou
- un groupement -(CH2 )a - (O-$C_x$H2$_x$m-(O-$C_y$H2$_y$)n- O-$R_{16}$ dans lequel

  - a est un nombre entier allant de 0 à 8,
  - $R_{16}$ représente un atome d'hydrogène ou un radical alkyle en C1-C6, tel que méthyle, éthyle, propyle, butyle, pentyle ou hexyle, avec la condition que l'un au moins des radicaux $R_{13}$, $R_{14}$ et $R_{15}$, est un groupement -(CH2 )a - (O$C_x$H2$_x$)m-(O-$C_y$H2$_y$)n - O-$R_{16}$ tel que défini ci-dessus,
  - x et y, identiques ou différents, sont des entiers allant de 2 à 10, de préférence de 3 à 5, et mieux, x=3 et y=0,
  - m et n sont indépendamment des entiers allant de 0 à 1000, de préférence de 0 à 100,

- A est un nombre entier allant de 0 à 200 ;
- B est un nombre entier allant de 0 à 100 ; à la condition que A et B ne soient pas simultanément égaux à zéro.

**31.** Composition selon la revendication précédente, **caractérisée en ce que** :

- B=0,
- A est un entier allant de 0 à 200,
- $R_{13}$ et $R_{15}$ sont identiques et représentent un radical -(CH2 )a - (O-$C_x$H2$_x$)m-(O-CyH2y)n- O-$R_{16}$ dans lequel a est un nombre entier allant de 0 à 8, $R_{16}$ représente un atome d'hydrogène ou un radical alkyle en C1-C6, x=3 et y=0 et m et n sont indépendamment des entiers allant de 0 à 1000, de préférence de 0 à 100.

**32.** Composition selon l'une des revendications précédentes, **caractérisée en ce que** l'agent plastifiant est un dimethicone copolyol à groupements α, ω propyl polyoxypropylène.

**33.** Composition selon l'une des revendications précédentes, **caractérisée en ce que** l'agent plastifiant est présent en une quantité allant de 1 à 15% en poids par rapport au poids total de la composition, de préférence de 2 à 10%, et mieux de 3 à 8% en poids.

**34.** Composition selon rune des revendications précédentes, **caractérisée en ce qu'**elle comprend une matière colorante.

**35.** Composition selon la revendication précédente, **caractérisée en ce que** la matière colorante est présent en une teneur allant de 0,01 % à 50 % en poids, par rapport au poids de la composition, de préférence de 0,01 % à 30 % en poids.

**36.** Procédé cosmétique de maquillage ou de soin non thérapeutique des ongles comprenant l'application sur les ongles d'au moins une couche d'une composition de vernis à ongles selon l'une des revendications 1 à 35.

**37.** Utilisation d'une composition selon l'une des revendications 1 à 35 pour obtenir un film, déposé sur les ongles, brillant, de bonne tenue et résistant à l'eau.

**38.** Ensemble cosmétique comprenant : i) un récipient délimitant au moins un compartiment, ledit récipient étant fermé par un élément de fermeture et ii) une composition disposée à l'intérieur dudit compartiment, la composition étant conforme à l'une quelconque des revendications 1 à 35.

## Claims

**1.** Nail varnish composition comprising, in a cosmetically acceptable medium comprising an organic solvent medium, at least one block polymer and at least one plasticizer selected from esters obtained from the reaction of a carboxylic acid with a diol, polyethers, dimethicone copolyols, ethyltosylamide and mixtures thereof,
the said block polymer comprising at least one first block and at least one second block, the said first and second blocks being linked together via an intermediate segment comprising at least one constituent monomer of the first block and at least one constituent monomer of the second block,
the first block having a glass transition temperature (Tg) greater than or equal to 40°C, the said first block being totally or partially derived from one or more monomers which are such that the homopolymer prepared from these monomers has a glass transition temperature of greater than or equal to 40°C, or the said first block being a copolymer derived from monomers which are such that the homopolymer prepared from these monomers has a glass transition temperature of greater than or equal to 40°C,
the monomers whose corresponding homopolymer has a glass transition temperature of greater than or equal to 40°C being chosen from the following monomers:

- methacrylates of formula $CH_2$= $C(C_H3)$-$COOR_1$
in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group,
- acrylates of formula $CH_2$ = CH-$COOR_2$ in which $R_2$ represents a $C_4$ to $C_{12}$ cycloalkyl group such as isobornyl acrylate or a tert-butyl group,
- and mixtures thereof,
and the second block having a glass transition temperature of less than or equal to 20°C, the said second block being a homopolymer derived from monomers which are such that the homopolymer prepared from these monomers has a glass transition temperature of less than or equal to 20°C,

the monomers whose corresponding homopolymer has a glass transition temperature of less than or equal to 20°C being chosen from the following monomers:

- acrylates of formula $CH_2$ = $CHCOOR_3$,
$R_3$ representing a linear or branched $C_1$ to $C_{12}$ unsubstituted alkyl group, with the exception of the tert-butyl group, in which one or more hetero atoms chosen from 0, N and S is (are) optionally intercalated;
- methacrylates of formula $CH_2$ = $C(CH_3)$-$COOR_4$,
$R_4$ representing a linear or branched $C_6$ to $C_{12}$ unsubstituted alkyl group, in which one or more hetero atoms chosen from O, N and S is (are) optionally intercalated;
- and mixtures thereof.

**2.** Composition according to the preceding claim, **characterized in that** the block polymer is a film-forming linear ethylenic block polymer.

**3.** Composition according to either of Claims 1 and 2, **characterized in that** the monomers whose corresponding homopolymer has a glass transition temperature of greater than or equal to 40°C are chosen from methyl methacrylate, isobutyl methacrylate and isobornyl (meth)acrylate, and mixtures thereof.

**4.** Composition according to one of Claims 1 to 3, **characterized in that** the proportion of the first block of the block polymer ranges from 20% to 90% by weight, better still from 30% to 80% and even better still from 50% to 70% by weight of the polymer.

**5.** Composition according to one of Claims 1 to 4, **characterized in that** the monomers whose corresponding homopolymer has a glass transition temperature of less than or equal to 20°C are chosen from alkyl acrylates whose alkyl chain contains from 1 to 10 carbon atoms, with the exception of the tert-butyl group.

**6.** Composition according to one of Claims 1 to 5, **characterized in that** the proportion of the second block with a Tg

of less than or equal to 20°C of the block polymer ranges from 5% to 75% by weight, better still from 15% to 50% and even better still from 25% to 45% by weight of the polymer.

7. Composition according to one of the preceding claims, **characterized in that** the first block and/or the second block of the block polymer comprises at least one additional monomer.

8. Composition according to the preceding claim, **characterized in that** the additional monomer is chosen from hydrophilic monomers and ethylenically unsaturated monomers comprising one or more silicon atoms, and mixtures thereof.

9. Composition according to Claim 7 or 8, **characterized in that** the additional monomer is chosen from:

- ethylenically unsaturated monomers comprising at least one carboxylic or sulphonic acid function,
- methacrylates of formula $CH_2 = C(CH_3)\text{-}COOR_6$ in which $R_6$ represents a linear or branched alkyl group containing from 1 to 4 carbon atoms, such as a methyl, ethyl, propyl or isobutyl group, the said alkyl group being substituted with one or more substituents chosen from hydroxyl groups and halogen atoms,
- methacrylates of formula $CH_2 = C(C_H3)\text{-}COOR_9$, $R_9$ representing a linear or branched $C_6$ to $C_{12}$ alkyl group in which one or more hetero atoms chosen from 0, N and S is (are) optionally intercalated, the said alkyl group being substituted with one or more substituents chosen from hydroxyl groups and halogen atoms (Cl, Br, I or F);
- acrylates of formula $CH_2 = CHCOOR_{10}$,
$R_{10}$ representing a linear or branched $C_1$ to $C_{12}$ alkyl group substituted with one or more substituents chosen from hydroxyl groups and halogen atoms (C1, Br, I or F), such as 2-hydroxypropyl acrylate and 2-hydroxyethyl acrylate, or $R_{10}$ represents a $C_1$ to $C_{12}$ alkyl-O-POE (polyoxyethylene) with repetition of the oxyethylene unit 5 to 30 times, for example methoxy-POE, or $R_{10}$ represents a polyoxyethylenated group comprising from 5 to 30 ethylene oxide units,
- ethylenically unsaturated monomers comprising at least one tertiary amine functional group,
- and mixtures thereof.

10. Composition according to one of Claims 7 to 9, **characterized in that** the additional monomer(s) is(are) chosen from acrylic acid, methacrylic acid, trifluoroethyl methacrylate and mixtures thereof.

11. Composition according to one of Claims 7 to 10, **characterized in that** the additional monomer(s) represent(s) from 1 to 30% by weight of the total weight of the first and/or second blocks of the block polymer.

12. Composition according to one of the preceding claims, **characterized in that** each of the first and second block of the block polymer comprises at least one monomer chosen from (meth)acrylic acid esters and optionally at least one monomer chosen from (meth)acrylic acid, and mixtures thereof.

13. Composition according to one of the preceding claims, **characterized in that** each of the first and second block of the block polymer is totally derived from at least one monomer chosen from acrylic acid, (meth)acrylic acid esters and optionally from at least one monomer chosen from (meth)acrylic acid, and mixtures thereof.

14. Composition according to one of the preceding claims, **characterized in that** the difference between the glass transition temperatures (Tg) of the first and second blocks of the block polymer is greater than 30°C and better still greater than 40°C.

15. Composition according to one of the preceding claims, **characterized in that** the intermediate segment of the block polymer has a glass transition temperature between the glass transition temperatures of the first and second blocks.

16. Composition according to one of the preceding claims, **characterized in that** the block polymer has a polydispersity index I of greater than 2, preferably of greater than or equal to 2.5, preferably of greater than or equal to 2.8.

17. Composition according to one of the preceding claims, **characterized in that** the block polymer has a polydispersity index of between 2.8 and 6.

18. Composition according to one of the preceding claims, **characterized in that** the block polymer is not soluble at an active material content of at least 1% by weight in water or in a mixture of water and linear or branched lower monoalcohols having from 2 to 5 carbon atoms, without modification of pH, at room temperature (25°C).

**19.** Composition according to one of the preceding claims, **characterized in that** the block polymer is not an elastomer.

**20.** Composition according to one of the preceding claims, **characterized in that** the block polymer represents from 0.1 to 60% by weight in terms of dry matter relative to the total weight of the composition, preferably from 0.5 to 50% and more preferably from 1 to 40% by weight.

**21.** Composition according to one of the preceding claims, **characterized in that** the organic solvent medium comprises at least one organic solvent selected from:

- ketones which are liquid at ambient temperature, such as methyl ethyl ketone, methyl isobutyl ketone, diisobutyl ketone, isophorone, cyclohexanone and acetone;
- alcohols which are liquid at ambient temperature, such as ethanol, isopropanol, diacetone alcohol, 2-butoxyethanol and cyclohexanol;
- propylene glycol ethers which are liquid at ambient temperature, such as propylene glycol monomethyl ether, propylene glycol monomethyl ether acetate and dipropylene glycol mono-n-butyl ether;
- cyclic ethers such as γ-butyrolactone;
- short-chain esters (having 3 to 8 carbon atoms in total) such as ethyl acetate, methyl acetate, propyl acetate, isopropyl acetate, n-butyl acetate, isopentyl acetate, methoxypropyl acetate and butyl lactate;
- ethers which are liquid at ambient temperature, such as diethyl ether, dimethyl ether or dichlorodiethyl ether;
- alkanes which are liquid at ambient temperature, such as decane, heptane, dodecane or cyclohexane;
- alkyl sulphoxides such as dimethyl sulphoxide;
- aldehydes which are liquid at ambient temperature, such as benzaldehyde and acetaldehyde;
- ethyl 3-ethoxypropionate;
- carbonates such as propylene carbonate and dimethyl carbonate;
- acetals such as methylal;
- and mixtures thereof.

**22.** Composition according to one of the preceding claims, **characterized in that** the organic solvent medium represents from 10% to 95% by weight, relative to the total weight of the composition, preferably from 15% to 80% by weight and more preferably from 20% to 60% by weight.

**23.** Composition according to one of Claims 1 to 22, **characterized in that** the ester of carboxylic acid and diol is a monoester.

**24.** Composition according to one of Claims 1 to 23, **characterized in that** the ester of carboxylic acid and diol is obtained from reaction of a monocarboxylic acid of formula $R_{11}COOH$ with a diol of formula $HOR_{12}OH$ where $R_{11}$ and $R_{12}$, which are identical or different, represent a linear, branched or cyclic, saturated or unsaturated hydrocarbon chain containing preferably 3 to 15 carbon atoms and optionally one or more heteroatoms such as N, 0 and S.

**25.** Composition according to the preceding claim, **characterized in that** $R_{11}$ is a $C_3$-$C_5$ alkyl radical and $R_{12}$ is a saturated linear hydrocarbon chain containing 5 to 10 carbon atoms.

**26.** Composition according to one of the preceding claims, **characterized in that** the plasticizer is a monoester resulting from the reaction of isobutyric acid and 2,2,4-trimethylpentane-1,3-diol.

**27.** Composition according to one of the preceding claims, **characterized in that** the polyethers have the following general formula:

$$H\text{-} (O\text{-}C_xH_{2x})_m\text{-} (O\text{-}C_yH_{2y})_n\text{-}OH \qquad (I)$$

in which x and y, which are identical or different, are integers ranging from 2 to 10, preferably from 3 to 5, and m and n are, independently, integers ranging from 0 to 1000, preferably from 0 to 100.

**28.** Composition according to one of the preceding claims, **characterized in that** the polyethers are selected from polyethylene glycols, polypropylene glycols, polyethylene glycol-polypropylene glycol copolymers and mixtures thereof.

**29.** Composition according to one of the preceding claims, **characterized in that** the polyethers are selected from

polyethylene glycols and polypropylene glycols having a molecular mass ranging from 500 to 15 000, preferably from 600 to 10 000, and mixtures thereof.

**30.** Composition according to one of the preceding claims, **characterized in that** the dimethicone copolyols conform to the following formula (II):

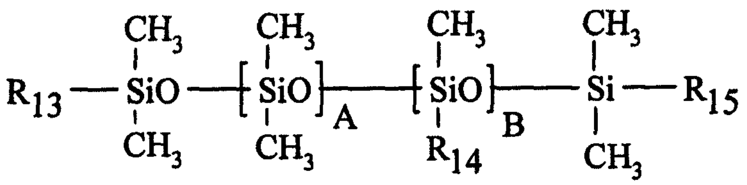

(II)

in which:

- $R_{13}$, $R_{14}$ and $R_{15}$ represent independently:
- a C1-C6 alkyl radical, such as methyl, ethyl, propyl, butyl, pentyl or hexyl, or
- a group $-(CH_2)_a-(O-C_xH_{2x})_m-(O-CyH_2y)_n-O-R_{16}$ in which

    - a is an integer ranging from 0 to 8,
    - $R_{16}$ represents a hydrogen atom or a C1-C6 alkyl radical, such as methyl, ethyl, propyl, butyl, pentyl or hexyl, on condition that at least one of the radicals $R_{13}$, $R_{14}$ and $R_{15}$ is a group - $(CH_2)_a$- $(O-C_XH_{2X})_m$- $(O-CyH_{2y})_n-O-R_{16}$ as defined above,
    - x and y, which are identical or different, are integers ranging from 2 to 10, preferably from 3 to 5, and more preferably, x=3 and y=0,
    - m and n are, independently, integers ranging from 0 to 1000, preferably from 0 to 100,

- A is an integer ranging from 0 to 200;
- B is an integer ranging from 0 to 100, with the proviso that A and B are not simultaneously zero.

**31.** Composition according to the preceding claim, **characterized in that**:

- B=0,
- A is an integer ranging from 0 to 200,
- $R_{13}$ and $R_{15}$ are identical and represent a radical $-(CH_2)_a-(O-C_xH_{2x})_m-(O-CyH_2y)_n-O-R_{16}$ in which a is an integer ranging from 0 to 8, $R_{16}$ represents a hydrogen atom or a C1-C6 alkyl radical, x=3 and y=0 and m and n are, independently, integers ranging from 0 to 1000, preferably from 0 to 100.

**32.** Composition according to one of the preceding claims, **characterized in that** the plasticizer is a dimethicone copolyol containing $\alpha,\omega$-propyl polyoxypropylene groups.

**33.** Composition according to one of the preceding claims, **characterized in that** the plasticizer is present in an amount ranging from 1% to 15% by weight relative to the total weight of the composition, preferably from 2% to 10% and more preferably from 3% to 8% by weight.

**34.** Composition according to one of the preceding claims, **characterized in that** it comprises a colorant.

**35.** Composition according to the preceding claim, **characterized in that** the colorant is present in an amount ranging from 0.01% to 50% by weight, relative to the weight of the composition, preferably from 0.01% to 30% by weight.

**36.** Cosmetic process for making up or non-therapeutically caring for the nails, comprising application to the nails of at least one layer of a nail varnish composition according to one of Claims 1 to 35.

**37.** Use of a composition according to one of Claims 1 to 35 to give a film, applied to the nails, which is glossy, has good holding power and is water-resistant.

**38.** Cosmetic assembly comprising: i) a container delimiting at least one compartment, the said container being closed by a closing member and ii) a composition placed inside the said compartment, the composition being in accordance with any one of Claims 1 to 35.

## Patentansprüche

**1.** Nagellack, der in einem kosmetisch akzeptablen Medium, das ein organisches Lösungsmittelmedium ist, mindestens ein Sequenzpolymer und mindestens einen Weichmacher enthält, der ausgewählt ist unter aus der Umsetzung einer Carbonsäure mit einem Diol resultierenden Estern, Polyethern, Dimethicon-Copolyolen und Ethyltosylamid sowie Gemischen dieser Verbindungen,
wobei das Sequenzpolymer mindestens eine erste Sequenz und mindestens eine zweite Sequenz aufweist und die erste Sequenz und die zweite Sequenz durch ein Zwischensegment miteinander verbunden sind, das mindestens ein Monomer, mit dem als Baustein die erste Sequenz aufgebaut ist, und mindestens ein Monomer enthält, mit dem als Baustein die zweite Sequenz aufgebaut ist,
wobei die erste Sequenz eine Glasübergangstemperatur (Tg) von 40 °C oder höher aufweist und wobei die erste Sequenz vollständig oder teilweise von einem oder mehreren Monomeren stammt, die so beschaffen sind, dass das aus diesen Monomeren hergestellte Homopolymer eine Glasübergangstemperatur von 40 °C oder höher aufweist, oder ein Copolymer ist, das von Monomeren stammt, die so beschaffen sind, dass das aus diesen Monomeren hergestellte Homopolymer eine Glasübergangstemperatur von 40 °C oder höher aufweist,
wobei die Monomeren, deren Homopolymer einer Glasübergangstemperatur von 40 °C oder höher entspricht, ausgewählt sind unter den nachstehenden Monomeren:

- Methacrylaten der Formel $CH_2=C(CH_3)-COOR_1$,
in der $R_1$ eine unsubstituierte, geradkettige oder verzweigte Alkylgruppe mit 1 bis 4 Kohlenstoffatomen bedeutet, wie etwa eine Gruppe Methyl, Ethyl, Propyl oder Isobutyl, oder $R_1$ eine $C_4$-$C_{12}$-Cycloalkylgruppe bedeutet,
- Acrylaten der Formel $CH_2=CH-COOR_2$,
worin $R_2$ eine $C_4$-$C_{12}$-Cycloalkylgruppe, wie im Fall von Isobornylacrylat, oder eine tert.-Butylgruppe bedeutet,
- und Gemischen dieser Verbindungen,

und wobei die zweite Sequenz eine Glasübergangstemperatur von 20 °C oder weniger aufweist und wobei die zweite Sequenz ein Homopolymer ist, das von Monomeren stammt, die so beschaffen sind, dass das aus diesen Monomeren hergestellte Homopolymer eine Glasübergangstemperatur von 20 °C oder weniger aufweist,
wobei die Monomeren, deren Homopolymer einer Glasübergangstemperatur von 20 °C oder weniger entspricht, ausgewählt sind unter den nachstehenden Monomeren:

- Acrylaten der Formel $CH_2=CHCOOR_3$,
worin $R_3$ eine unsubstituierte, geradkettige oder verzweigte $C_1$-$C_{12}$-Alkylgruppe, ausgenommen tert.-Butyl, bedeutet, in der gegebenenfalls eine oder mehrere unter O, N und S ausgewählte Heteroatome eingeschaltet sind,
- Methacrylaten der Formel $CH_2=C(CH_3)-COOR_4$,
in der $R_4$ eine unsubstituierte, geradkettige oder verzweigte $C_6$-$C_{12}$-Alkylgruppe bedeutet, in der gegebenenfalls ein oder mehrere unter O, N und S ausgewählte Heteroatome eingeschaltet sind,
sowie Gemischen dieser Verbindungen.

**2.** Zusammensetzung nach dem vorhergehenden Anspruch, **dadurch gekennzeichnet, dass** das Sequenzpolymer ein filmbildendes lineares Ethylen-Sequenzpolymer ist.

**3.** Zusammensetzung nach Anspruch 1 oder 2, **dadurch gekennzeichnet, dass** die Monomeren, deren Homopolymer einer Glasübergangstemperatur von 40 °C oder höher entspricht, unter Methylmethacrylat, Isobutylmethacrylat und Isobornyl(meth)acrylat sowie Gemischen dieser Verbindungen ausgewählt sind.

**4.** Zusammensetzung nach einem der Ansprüche 1 bis 3, **dadurch gekennzeichnet, dass** der Mengenanteil der ersten Sequenz des Sequenzpolymers 20 bis 90 Gew.-% des Polymers, günstiger 30 bis 80 Gew.-% und noch günstiger 50 bis 70 Gew.-% beträgt.

**5.** Zusammensetzung nach einem der Ansprüche 1 bis 4, **dadurch gekennzeichnet, dass** die Monomeren, deren Homopolymer einer Glasübergangstemperatur von 20 °C oder weniger entspricht, unter Alkylacrylaten ausgewählt

sind, deren Alkylkette 1 bis 10 Kohlenstoffatome aufweist, wobei die tert.-Butylgruppe ausgenommen ist.

6. Zusammensetzung nach einem der Ansprüche 1 bis 5, **dadurch gekennzeichnet, dass** der Mengenanteil der zweiten Sequenz des Sequenzpolymers, die einen Wert Tg von 20 °C oder weniger aufweist, 5 bis 75 Gew.-% des Polymers, günstiger 15 bis 50 Gew.-% und noch günstiger 25 bis 45 Gew.-% beträgt.

7. Zusammensetzung nach einem der vorhergehenden Ansprüche, **dadurch gekennzeichnet, dass** die erste Sequenz und/oder die zweite Sequenz des Sequenzpolymers mindestens ein zusätzliches Monomer enthält.

8. Zusammensetzung nach dem vorhergehenden Anspruch, **dadurch gekennzeichnet, dass** das zusätzliche Monomer ausgewählt ist unter hydrophilen Monomeren und ethylenisch ungesättigten Monomeren, die ein oder mehrere Siliciumatome aufweisen, sowie Gemischen dieser Verbindungen.

9. Zusammensetzung nach Anspruch 7 oder 8, **dadurch gekennzeichnet, dass** das zusätzliche Monomer ausgewählt ist unter

   - Monomeren mit einer oder mehreren ethylenisch ungesättigten Bindungen, die mindestens eine Carbonsäurefunktion oder Sulfonsäurefunktion aufweisen;
   - Methacrylaten der Formel $CH_2=C(CH_3)-COOR_6$,
   in der $R_6$ eine geradkettige oder verzweigte Alkylgruppe mit 1 bis 4 Kohlenstoffatomen bedeutet, wie beispielsweise eine Gruppe Methyl, Ethyl, Propyl oder Isobutyl, wobei die Alkylgruppe mit einem oder mehreren Substituenten substituiert ist, die unter Hydroxylgruppen und Halogenatomen ausgewählt sind;
   - Methacrylaten der Formel $CH_2=C(CH_3)-COOR_9$,
   wobei $R_9$ eine geradkettige oder verzweigte $C_6$-$C_{12}$-Alkylgruppe bedeutet, in der ein oder mehrere Heteroatome eingeschaltet sind, die unter O, N und S ausgewählt sind, wobei die Alkylgruppe mit einem oder mehreren Substituenten substituiert ist, die unter Hydroxylgruppen und Halogenatomen (Cl, Br, I, F) ausgewählt sind;
   - Acrylaten der Formel $CH_2=CHCOOR_{10}$,
   wobei $R_{10}$ eine geradkettige oder verzweigte $C_1$-$C_{12}$-Alkylgruppe bedeutet, die mit einem oder mehreren Substituenten substituiert ist, die unter Hydroxylgruppen und Halogenatomen (Cl, Br, I und F) ausgewählt sind, wobei es sich zum Beispiel um 2-Hydroxypropylacrylat und 2-Hydroxyethylacrylat handelt, oder $R_{10}$ $C_1$-$C_{12}$-Alkyl-O-POE (Polyoxyethylen) mit 5 bis 30 Oxyethyleneinheiten darstellt, zum Beispiel Methoxy-POE, oder $R_{10}$ eine Polyoxyethylen-Gruppierung mit 5 bis 30 Ethylenoxideinheiten bedeutet;
   - Monomeren mit einer oder mehreren ethylenisch ungesättigten Bindungen, die mindestens eine tertiäre Aminfunktion aufweisen,

   sowie Gemischen dieser Verbindungen.

10. Zusammensetzung nach einem der Ansprüche 7 bis 9, **dadurch gekennzeichnet, dass** das zusätzliche Monomer oder die zusätzlichen Monomeren ausgewählt sind unter Acrylsäure, Methacrylsäure und Trifluorethylmethacrylat sowie Gemischen dieser Verbindungen.

11. Zusammensetzung nach einem der Ansprüche 7 bis 10, **dadurch gekennzeichnet, dass** das zusätzliche Monomer oder die zusätzlichen Monomeren 1 bis 30 Gew.-% des Gesamtgewichts der ersten Sequenz und/oder der zweiten Sequenz des Sequenzpolymers ausmachen.

12. Zusammensetzung nach einem der vorhergehenden Ansprüche, **dadurch gekennzeichnet, dass** sowohl die erste Sequenz als auch die zweite Sequenz des Sequenzpolymers enthält: mindestens ein Monomer, das unter Estern von (Meth)acrylsäure ausgewählt ist, und gegebenenfalls mindestens ein Monomer, das unter (Meth)acrylsäure ausgewählt ist, sowie Gemische dieser Monomeren.

13. Zusammensetzung nach einem der vorhergehenden Ansprüche, **dadurch gekennzeichnet, dass** sowohl die erste Sequenz als auch die zweite Sequenz des Sequenzpolymers vollständig stammt von mindestens einem unter Acrylsäure und Estern von (Meth)acrylsäure ausgewähltem Monomer und gegebenenfalls mindestens einem unter (Meth)acrylsäure ausgewähltem Monomer sowie Gemischen dieser Monomeren.

14. Zusammensetzung nach einem der vorhergehenden Ansprüche, **dadurch gekennzeichnet, dass** der Abstand zwischen der Glasübergangstemperatur (Tg) der ersten Sequenz und der Glasübergangstemperatur (Tg) der zweiten Sequenz des Sequenzpolymers mehr als 30 °C und noch günstiger mehr als 40 °C beträgt.

**15.** Zusammensetzung nach einem der vorhergehenden Ansprüche, **dadurch gekennzeichnet, dass** die Zwischensegment des Sequenzpolymers eine Glasübergangstemperatur aufweist, die zwischen den Glasübergangstemperaturen der ersten Sequenz und der zweiten Sequenz liegt.

**16.** Zusammensetzung nach einem der vorhergehenden Ansprüche, **dadurch gekennzeichnet, dass** das Sequenzpolymer einen Polydispersitätsindex (I) von höher als 2, vorzugsweise von 2,5 oder höher und noch bevorzugter von 2,8 oder höher aufweist.

**17.** Zusammensetzung nach einem der vorhergehenden Ansprüche, **dadurch gekennzeichnet, dass** das Sequenzpolymer einen Polydispersitätsindex von 2,8 bis 6 aufweist.

**18.** Zusammensetzung nach einem der vorhergehenden Ansprüche, **dadurch gekennzeichnet, dass** das Sequenzpolymer nicht zu einem Wirkstoffgehalt von mindestens 1 Gew.-% in Wasser oder einem Gemisch von Wasser mit geradkettigen oder verzweigten niederen einwertigen Alkoholen mit 2 bis 5 Kohlenstoffatomen ohne Änderung des pH-Werts bei Umgebungstemperatur (25 °C) löslich ist.

**19.** Zusammensetzung nach einem der vorhergehenden Ansprüche, **dadurch gekennzeichnet, dass** das Sequenzpolymer kein Elastomer ist.

**20.** Zusammensetzung nach einem der vorhergehenden Ansprüche, **dadurch gekennzeichnet, dass** das Sequenzpolymer 0,1 bis 60 Gew.-% der Trockensubstanz, bezogen auf das Gesamtgewicht der Zusammensetzung, und vorzugsweise 0,5 bis 50 Gew.-% und noch günstiger 1 bis 40 Gew.-% der Trockensubstanz ausmacht.

**21.** Zusammensetzung nach einem der vorhergehenden Ansprüche, **dadurch gekennzeichnet, dass** das organische Lösungsmittelmedium mindestens ein organisches Lösungsmittel enthält, das ausgewählt ist unter:

- bei Umgebungstemperatur flüssigen Ketonen, wie Methylethylketon, Methylisobutylketon, Diisobutylketon, Isophoron, Cyclohexanol und Aceton;
- bei Umgebungstemperatur flüssigen Alkoholen, wie Ethanol, Isopropanol, Diacetonalkohol, 2-Butoxyethanol und Cyclohexanol;
- bei Umgebungstemperatur flüssigen Ethern von Propylenglycol, wie Propylenglycolmonomethylether, Propylenglycolmonomethyletheracetat und Dipropylenglycolmono-n-butylether;
- cyclischen Ethern, wie γ-Butyrolacton;
- kurzkettigen Estern (mit insgesamt 3 bis 8 Kohlenstoffatomen), wie Ethylacetat, Methylacetat, Propylacetat, Isopropylacetat, n-Butylacetat, Isopentylacetat, Methoxypropylacetat und Butyllactat;
- bei Umgebungstemperatur flüssigen Ethern, wie Diethylether, Dimethylether oder Dichlordiethylether;
- bei Umgebungstemperatur flüssigen Alkanen, wie Decan, Heptan, Dodecan und Cyclohexan;
- Alkylsulfoxiden, wie Dimethylsulfoxid;
- bei Umgebungstemperatur flüssigen Aldehyden, wie Benzaldehyd und Acetaldehyd;
- 3-Ethoxypropionsäureethylester;
- Carbonaten, wie Propylencarbonat und Dimethylcarbonat;
- Acetalen, wie Methylal, sowie
- Gemischen dieser Verbindungen.

**22.** Zusammensetzung nach einem der vorhergehenden Ansprüche, **dadurch gekennzeichnet, dass** das organische Lösungsmittelmedium 10 bis 95 Gew.-% der Zusammensetzung, bezogen auf das Gesamtgewicht der Zusammensetzung, vorzugsweise 15 bis 80 Gew.-% und noch günstiger 20 bis 60 Gew.-% des Gesamtgewichts der Zusammensetzung ausmacht.

**23.** Zusammensetzung nach einem der Ansprüche 1 bis 22, **dadurch gekennzeichnet, dass** der Ester einer Carbonsäure mit einem Diol ein Monoester ist.

**24.** Zusammensetzung nach einem der Ansprüche 1 bis 23, **dadurch gekennzeichnet, dass** der Ester einer Carbonsäure mit einem Diol aus der Umsetzung einer Monocarbonsäure der Formel $R_{11}COOH$ mit einem Diol der Formel $HOR_{12}OH$ stammt, wobei $R_{11}$ und $R_{12}$, die gleich oder verschieden sind, eine geradkettige, verzweigte oder cyclische und gesättigte oder ungesättigte Kohlenwasserstoffkette mit vorzugsweise 3 bis 15 Kohlenstoffatomen bedeuten, die gegebenenfalls ein oder mehrere Heteroatome wie N, O und S enthält.

**25.** Zusammensetzung nach dem vorhergehenden Anspruch, **dadurch gekennzeichnet, dass** $R_{11}$ eine $C_3$-$C_5$-Alkylgruppe und $R_{12}$ eine gesättigte geradkettige Kohlenwasserstoffgruppe mit 5 bis 10 Kohlenstoffatomen darstellen.

**26.** Zusammensetzung nach einem der vorhergehenden Ansprüche, **dadurch gekennzeichnet, dass** der Weichmacher ein aus der Umsetzung von Isobuttersäure mit 2,2,4-Trimethylpentan-1,3-diol resultierender Monoester ist.

**27.** Zusammensetzung nach einem der vorhergehenden Ansprüche, **dadurch gekennzeichnet, dass** die Polyether die nachstehende allgemeine Formel

$$H\text{-}(O\text{-}C_xH2_x)_m\text{-}(O\text{-}C_yH_{2y})_n\text{-}OH \qquad (I)$$

aufweisen,
in der bedeuten:

x und y, die gleich oder verschieden sind, ganze Zahlen von 2 bis 10 und vorzugsweise von 3 bis 5, und
m und n unabhängig ganze Zahlen von 0 bis 1000 und
vorzugsweise von 0 bis 100.

**28.** Zusammensetzung nach einem der vorhergehenden Ansprüche, **dadurch gekennzeichnet, dass** die Polyether ausgewählt sind unter Polyethylenglycolen, Polypropylenglycolen und Polyethylenglycol-Polypropylenglycol-Copolymeren sowie Gemischen dieser Verbindungen.

**29.** Zusammensetzung nach einem der vorhergehenden Ansprüche, **dadurch gekennzeichnet, dass** die Polyether ausgewählt sind unter Polyethylenglycolen und Polypropylenglycolen mit einer Molekülmasse von 500 bis 15 000 und vorzugsweise von 600 bis 10 000 sowie Gemischen dieser Verbindungen.

**30.** Zusammensetzung nach einem der vorhergehenden Ansprüche, **dadurch gekennzeichnet, dass** die Dimethicon-Copolyole die nachstehende Formel (II) besitzen:

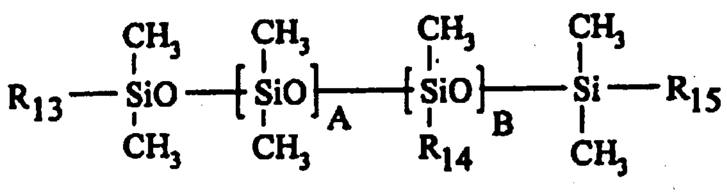

(II),

in der bedeuten:

- $R_{13}$, $R_{14}$ und $R_{15}$ unabhängig
  - eine $C_1$-$C_6$-Alkylgruppe, wie Methyl, Ethyl, Propyl, Butyl, Pentyl oder Hexyl, oder
  - eine Gruppe $-(CH_2)_a-(O-C_xH_{2x})_m-(O-C_yH_{2y})_n-O-R_{16}$, worin bedeuten:
    - a eine ganze Zahl von 0 bis 8,
    - $R_{16}$ ein Wasserstoffatom oder eine $C_1$-$C_6$-Alkylgruppe, wie Methyl, Ethyl, Propyl, Butyl, Pentyl oder Hexyl, mit der Maßgabe, dass mindestens eine der Gruppen $R_{13}$, $R_{14}$ und $R_{15}$ eine Gruppe $-(CH_2)_a-(O-C_xH_{2x})_m-(O-CyH_{2y})_n-O-R_{16}$ wie oben definiert ist,
    - x und y, die gleich oder verschieden sind, ganze Zahlen von 2 bis 10 und vorzugsweise von 3 bis 5, wobei noch günstiger x=3 und y=0 sind,
    - m und n unabhängig ganze Zahlen von 0 bis 1000 und vorzugsweise von 0 bis 100,
  - A eine ganze Zahl von 0 bis 200;
  - B eine ganze Zahl von 0 bis 100,

  mit der Maßgabe, dass A und B nicht gleichzeitig gleich Null sind.

**31.** Zusammensetzung nach dem vorhergehenden Anspruch, **dadurch gekennzeichnet, dass** bedeuten:

- B O,
- A eine ganze Zahl von 0 bis 200,
- $R_{13}$ und $R_{15}$, die identisch sind, eine Gruppe $-(CH_2)_a-(O-C_xH_{2x})_m-(O-CyH_{2y})_n-O-R_{16}$, worin bedeuten:
  - a eine ganze Zahl von 0 bis 8,
  - $R_{16}$ ein Wasserstoffatom oder eine $C_1$-$C_6$-Alkylgruppe,
  - x 3 und y 0 und m und n unabhängig ganze Zahlen von 0 bis 1000 und vorzugsweise von 0 bis 100.

**32.** Zusammensetzung nach einem der vorhergehenden Ansprüche, **dadurch gekennzeichnet, dass** der Weichmacher ein Dimethicon-Copolyol ist, das $\alpha,\omega$-Propylpolyoxypropylen-Gruppen aufweist.

**33.** Zusammensetzung nach einem der vorhergehenden Ansprüche, **dadurch gekennzeichnet, dass** der Weichmacher in einer Menge von 1 bis 15 Gew.-%, bezogen auf das Gesamtgewicht der Zusammensetzung, vorzugsweise in einer Menge von 2 bis 10 Gew.-% und noch günstiger in einer Menge von 3 bis 8 Gew.-% vorliegt.

**34.** Zusammensetzung nach einem der vorhergehenden Ansprüche, **dadurch gekennzeichnet, dass** sie einen Farbstoff enthält.

**35.** Zusammensetzung nach dem vorhergehenden Anspruch, **dadurch gekennzeichnet, dass** der Farbstoff in einem Mengenanteil von 0,01 bis 50 Gew.-%, bezogen auf das Gewicht der Zusammensetzung, und vorzugsweise in einem Mengenanteil von 0,01 bis 30 Gew.-%, vorliegt.

**36.** Kosmetisches Verfahren zum Make-up oder zur nichttherapeutischen Pflege der Nägel, welches das Aufbringen mindestens einer Schicht einer Nagellackzusammensetzung nach einem der Ansprüche 1 bis 35 auf die Nägel umfasst.

**37.** Verwendung einer Zusammensetzung nach einem der Ansprüche 1 bis 35 zur Erzielung eines auf die Nägel aufgebrachten glänzenden Films guter Haltbarkeit und Wasserfestigkeit.

**38.** Kosmetische Einheit, die aufweist: i) einen Behälter, der mindestens ein Compartment vorgibt, wobei der Behälter durch ein Verschlusselement verschlossen ist, und ii) eine im Inneren des Compartments vorgesehene Zusammensetzung nach einem der Ansprüche 1 bis 35.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0648485 A